# EUROPEAN PATENT SPECIFICATION

(11) **EP 1 343 418 B1**
(45) Date of publication and mention of the grant of the patent: **11.03.2009**
(21) Application number: 01990938.1
(22) Date of filing: 08.11.2001
(51) Int. Cl.: A61B 17/04

(54) **SURGICAL INSTRUMENT FOR TREATING FEMALE URINARY INCONTINENCE**
OPERATIONSINSTRUMENT ZUR BEHANDLUNG DER WEIBLICHEN HARNINKONTINENZ
INSTRUMENT CHIRURGICAL PERMETTANT DE TRAITER L'INCONTINENCE D'URINE CHEZ LA FEMME

(30) Priority: 20.11.2000 US 716546
(43) Date of publication of application: 17.09.2003
(73) Proprietor: ETHICON, INC., Somerville, NJ 08876 (US)
(72) Inventor: ULMSTEN, Ulf, S-S-182 35 Danderyd (SE); KAMMERER, Gene, W., East Brunswick, NJ 08816 (US)
(74) Representative: Mercer, Christopher Paul
(86) International application number: PCT/US2001/047416
(87) International publication number: WO 2002/039890

(56) References cited:
- EP-A- 0 852 930
- WO-A-00/74613
- WO-A-94/05213
- US-A- 3 038 475
- US-A- 3 608 095
- US-A- 5 368 595
- US-A- 5 450 860
- US-A- 5 899 909
- US-A- 5 935 122

## Description

### FIELD OF THE INVENTION

The invention relates to a surgical instrument for treating female urinary incontinence, that is, the incapacity of controlling the discharge of urine.

### BACKGROUND OF THE INVENTION

Urinary incontinence may be caused by a defect function in the tissue or ligaments connecting the vaginal wall with the pelvic muscles and pubic bone.

US Patent No. 5,112,344 describes a method for treating female urinary incontinence without the necessity of opening the abdomen, which would require extended hospital care. In this method a tape is looped around the muscle tissue of the abdomen to either side of urethra to be implanted into the soft tissue between the vaginal wall and the abdominal wall extending over pubis and with the ends of the tape extending into vagina. The tape is left in the body in order that fibrous tissue shall develop around the tape, said scar tissue functioning as a supporting ligament in the soft tissue. The tape is removed from the body when such scar tissue has developed, which takes about two months.

The result obtained by such surgery is not always satisfactory due to the fact that fibrous tissue will not develop sufficiency since the soft tissue between the vaginal wall and the abdominal wall is in bad condition.

US 5,899,909 discloses a surgical instrument for treating female urinary incontinence comprising a shank having a handle at one end and two curved needle-like elements. The needle-like elements are each connected to one end of a tape which is intended to be implanted into the body. The disclosed instrument configured to penetrate the vaginal wall, pass close to the back of the pubic bone and then through the abdominal wall above the pubic bone.

### SUMMARY OF THE INVENTION

The object of the invention is to provide improved and simplified surgery with a considerably improved prognosis with regard to restoration of the urinary continence. The invention is directed towards a surgical instrument for treating female urinary incontinence, as defined in claim 1. Preferred embodiments are defined in the appended claims.

For this purpose the invention provides a surgical instrument for treating female urinary incontinence of the kind referred to above, comprising a shank, a handle at one end of said shank, a tape to be permanently implanted into the body as a loop around the urethra, two curved needle-like elements which are each connected to opposite ends of the tape, and means on said shank and each of said elements for exchangeable connection of the elements one at the time to the shank at the other end thereof to form at said other end a curved end portion dimensioned to extend from the inside surface of the vaginal wall and pass in front of the pubic bone to the outside of the abdominal wall.

A method for treating female urinary incontinence is thus permitted, comprising the steps of passing a tape into the body via the vagina first at one end thereof and then at the other end thereof at one side and the other, respectively, of urethra to form a loop around urethra, located between urethra and the vaginal wall, extending said tape in front of the pubic bone and through the lower abdominal wall, the ends of the tape being available outside the abdominal wall, adjusting the tape, and leaving the tape implanted in the body. Preferably the tape is left permanently in the body to provide itself, as an artificial ligament, the reinforcement of the tissue required in order to restore the urinary continence, and/or to provide said reinforcement by the development of fibrous tissue.

The invention will be explained in more detail with reference to the accompanying drawings which disclose embodiments of the surgical instrument according to the invention as well as several surgical steps using said surgical instrument.

In the drawings:
FIG. 1 is an elevation view of the surgical instrument in one embodiment thereof,
FIG. 2 is a plan view of the surgical instrument of Fig. 1,
FIG. 3 is an enlarged fragmentary axial cross sectional view of a coupling of the instrument for attaching an exchangeable part thereof,
FIGS. 4 to 11 illustrate diagrammatically several surgical steps of the method using the surgical instrument of Fig. 1.
FIG. 12 is an elevation view of the surgical instrument in a second, preferred embodiment thereof,
FIG. 13 is a plan view of the surgical instrument disclosed in FIG. 12.
FIG. 14 is an exploded side view of one of the needles and tape and shrinkage hose to be connected with said needle,
FIG. 15 is a side view of the needle in FIG. 14 with the tape connected therewith.
FIG. 16 is an enlarged fragmentary axial cross sectional view of a modified coupling of the instrument for connecting exchangeable needles of the kind shown in FIGS 14 and 15, and
FIG. 17 is a side view of two needles and a tape interconnecting said needles.

### DETAILED DESCRIPTION OF THE INVENTION

Before explaining the present invention in detail, it should be noted that the invention is not limited in its application or use to the details of construction and arrangement of parts illustrated in the accompanying drawings and description, because the illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiment of the present invention.

The invention discloses an apparatus for treating SUI. A tape is passed through pelvic tissue and positioned underneath the urethra, creating a supportive sling. The tape provides a structure means for tissue ingrowth and thereby provides a newly created body tissue supporting means for the urethra. When pressure is exerted upon the lower abdomen, such as during a cough or sneeze, the tape provides support to the urethra, allowing it to keep its seal and prevent the unwanted discharge of urine.

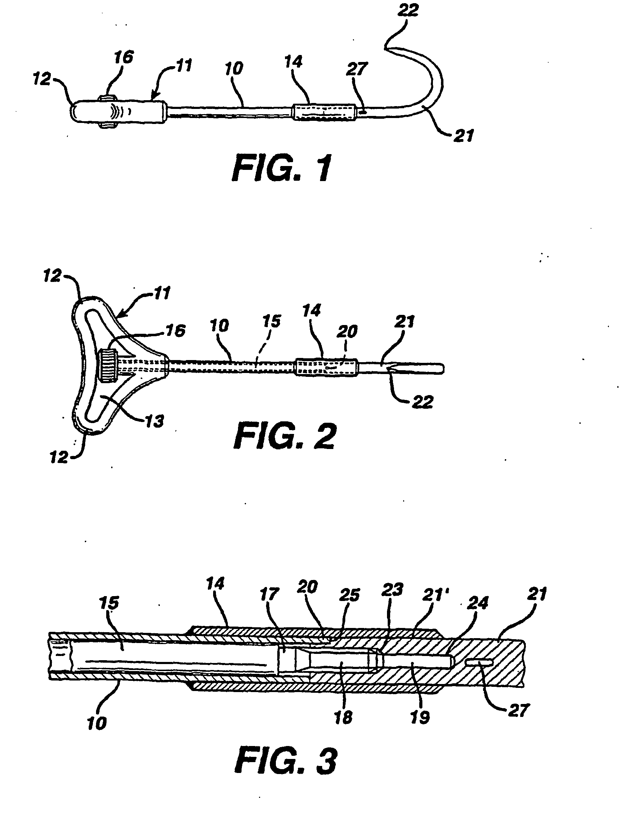

The surgical instrument of FIGS 1 to 3 comprises a cylindrical tubulary shank 10 having at one end thereof a handle 11 which forms two in opposite directions in a common plane projecting wings 12 and an opening 13. At the other end of the shank there is a socket 14 which is partly passed onto the shank and is soldered or brazed to the shank, a portion of the socket projecting from the shank at said other end thereof. A cylindrical shaft 15 is rotatably mounted in the shank and can be rotated manually by means of a knob 16 axially knurled at the outside surface thereof, which is mounted to one end of the shaft and is received by opening 13. The other end of the shaft forms a cylindrical portion 17 of smaller outside diameter than the shaft, which joins a portion 18 having external threads, a smooth end portion 19 of further reduced diameter joining the threaded portion 18, end portion 19 forming a guide pin at said other end of the shaft. Portions 18 and 19 are received in the portion of socket 14 projecting from the shank, and also a shoulder 20 projecting from the shank is received in said portion.

The surgical instrument as described so far is intended to be used several times and therefore should consist of a material which can be sterilized by autoclaving, e.g. of stainless steel.

The surgical instrument also includes an exchangeable and disposable element 21, which will be termed needle. It is attached to the shank at a straight portion 21' at one end of the needle and extends over substantially a half of a circle to the other, free end thereof in order to pass below the pubic bone from an access within the vagina. The needle has circular cross section and has a smooth, preferably polished outside surface. It tapers slightly towards the free end thereof where the needle forms a point 22 by being conical or, as shown, faceted but it can also be blunt-ended and have a transversely cut end. The practical use of the surgical instrument so far has shown that the conical shape of the point is preferred. The disposable needle shall be made either of a tissue compatible plastics, such as polycarbonate, or of steel or a similar material.

For attachment of needle 21 to shank 10 the needle has at said one end thereof where the needle forms said straight portion 21' to be received in socket 14, an axial blind hole extending from the end surface, said hold having a threaded portion 23 and inwardly thereof a narrower, cylindrical portion 24. Guide pin 19 is dimensioned to be guidingly received by said latter portion when the threaded portion 18 for attaching needle 21 to the rest of the surgical instrument is screwed into threaded portion 23 of the blind hole by rotating shaft 15 by manual rotation of knob 16, the end surfaces of the shank and the needle being pressed against each other. The needle should be oriented in a predetermined rotational position in relation to the shank; it should project at right angles to the plane of handle 11 and this rotational position is secured by shoulder 20 on the shank being received in a mating recess 25 in the outside surface of the needle.

Portion 23 of needle 21 instead of being threaded can be dimensioned such that the threaded portion 18 of shaft 15 cuts a thread in the material of the needle when being screwed thereinto.

When the two parts of the surgical instrument are screwed together in the manner described they form a rigid unit which can be controlled with great precision at handle 11 when it is used for surgery by applying the method of the invention.

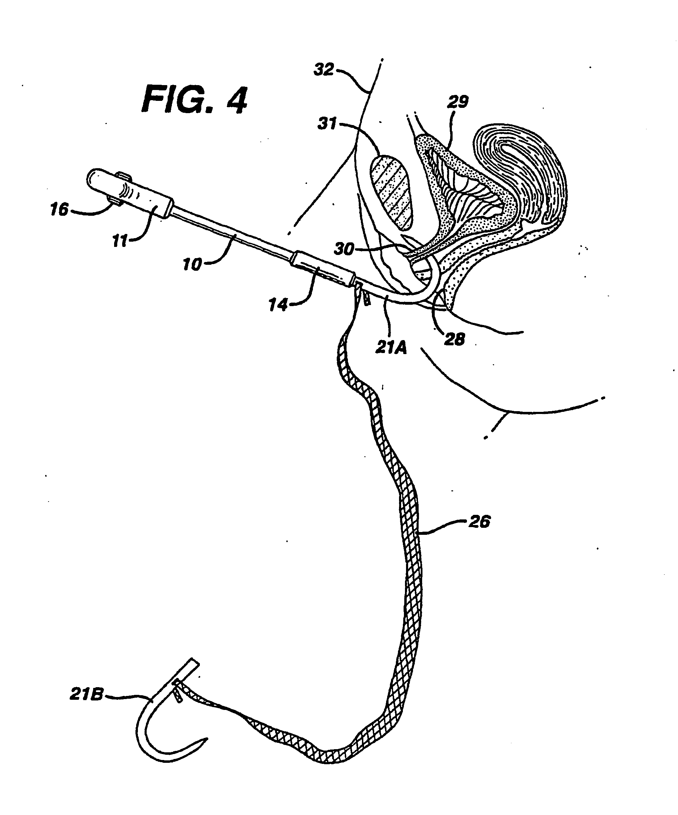

When the method is practiced, two needles 21A and 21 B of the embodiment described shall be connected one at each end of a tape 26, FIG. 4. The tape end can be glued to the needle but the connection can be effected also by the tape being passed through an eye 27, FIG. 3, in the needle adjacent the end attached to the shank or by the tape end being connected by ultrasonic welding to the needle or being baked into the plastics material of the needle at injection molding thereof.

When the surgery for implanting the tape shall start, one needle 21A is attached to shank 10, the other needle 21 B hanging loosely in tape 26 as shown in FIG. 4. In still a further embodiment, needle 21 may comprise quick attachment and detachment means to enable a single needle use as is disclosed in U.S. 2001 049467.

In FIGS. 4 to 11 the relevant parts of the female lower abdomen are disclosed diagrammatically, the vagina being designated 28, the urinary bladder 29, the urethra 30, the pubic bone 31, and the abdominal wall 32.

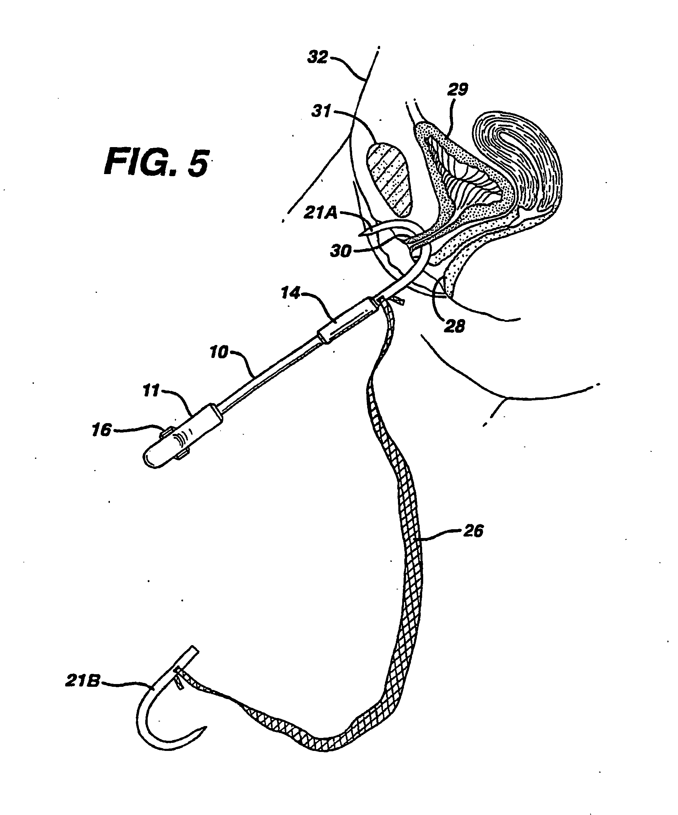

The first step of the surgery for implanting tape 26 is disclosed in FiG. 4 and comprises penetration of the vaginal wall by needle 21A, an incision having first been made in said wall, and also penetration of the soft tissue at one side of urethra 30, the needle then according to FIG. 5 being passed close to the underside of the pubic bone 31 and then through the lower abdominal wall 32. An incision can be made through the abdominal wall for the passage of the needle therethrough but if the needle is pointed it may be sufficient to let the needle penetrate into the abdominal wall from the inside thereof and to make a registering incision in the abdominal wall on the outside thereof.

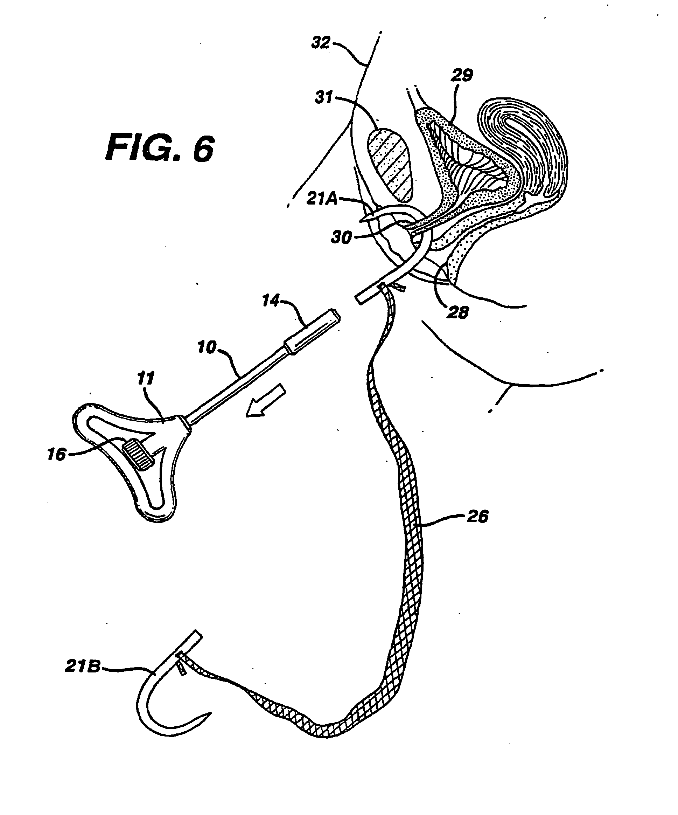

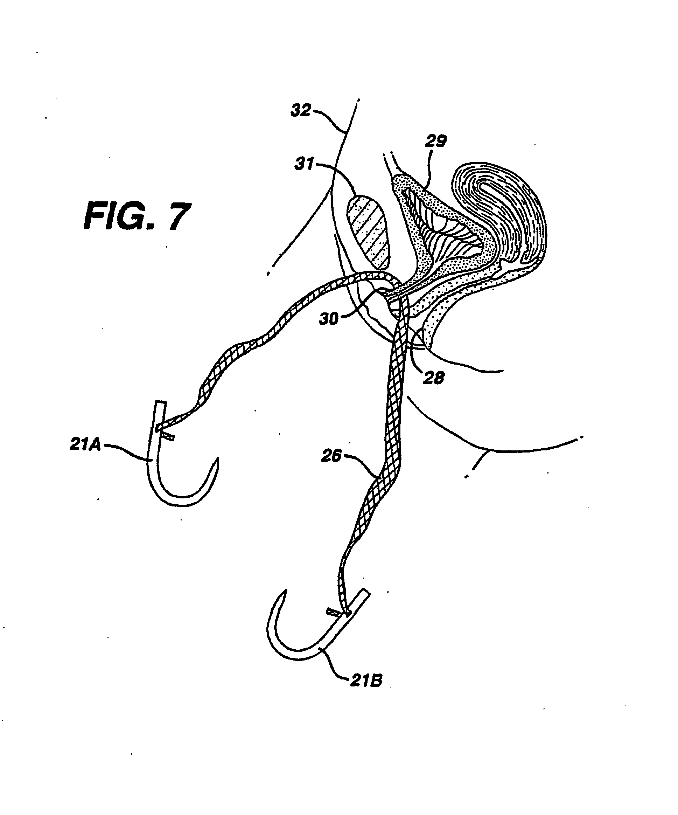

The shank of the instrument is now disconnected from needle 21A, FIG. 6, by rotating shaft 15 at knob 16 so that the threaded portion 18 of the shaft is unscrewed from the threaded portion 23 in needle 21A. Needle 21A is then withdrawn from the abdominal wall by means of forceps and tape 26 being pulled into and through the tissue as illustrated in FIG. 7.

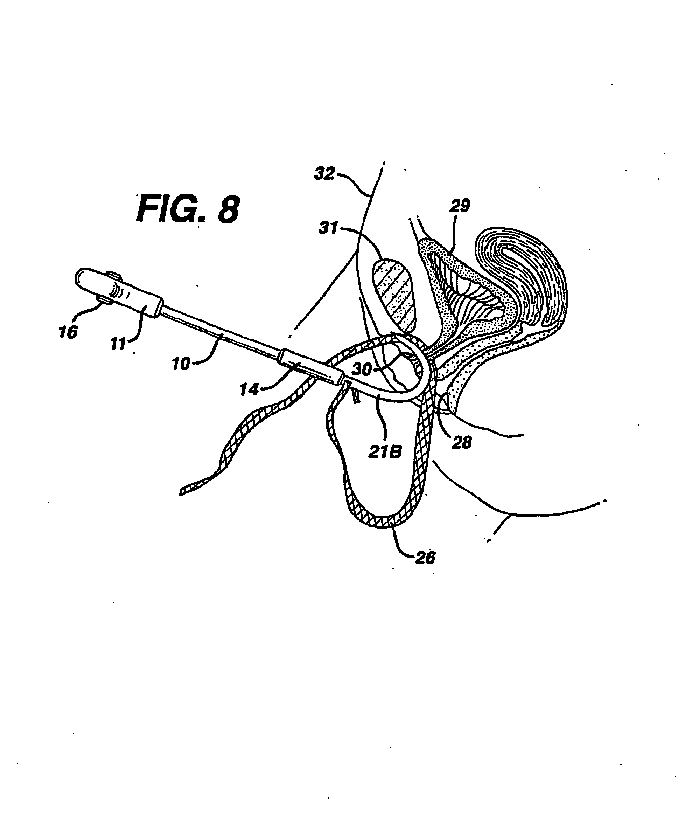

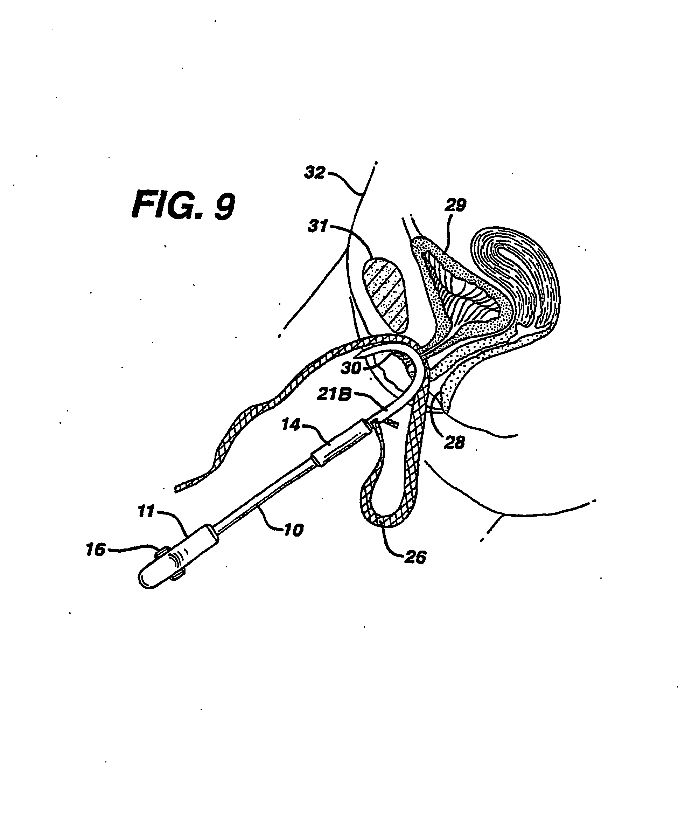

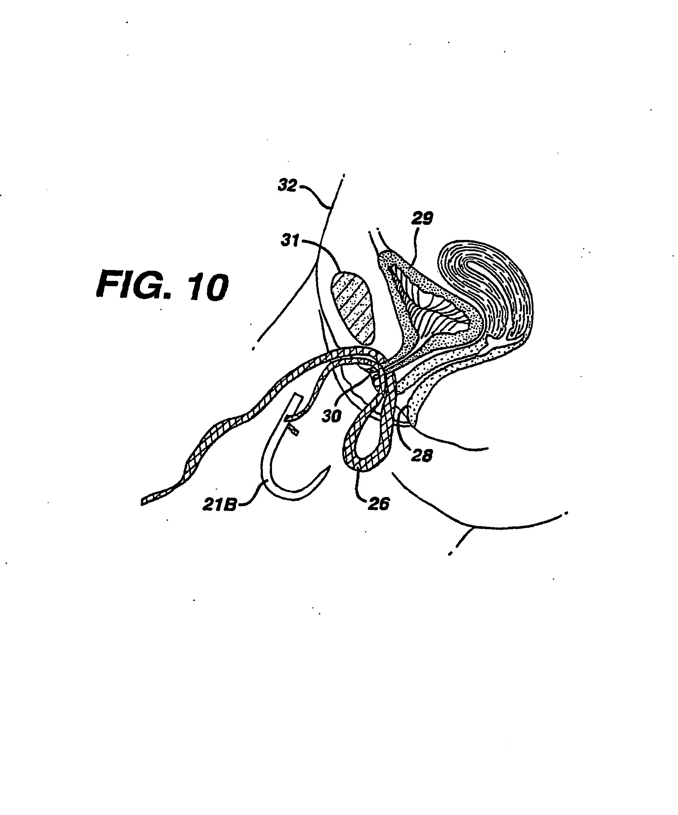

The other needle 21B is now attached to the shank, FIG. 8, and is passed through the incision in the vaginal wall to pass through the soft tissue at the other side of urethra 30. Needle 21B is passed below the pubic bone 31 and through the abdominal wall, FIG. 9, and then, after having been disconnected from the shank, is withdrawn from the abdominal wall, FIG. 10, all in the same way as in the earlier procedure with needle 21A.

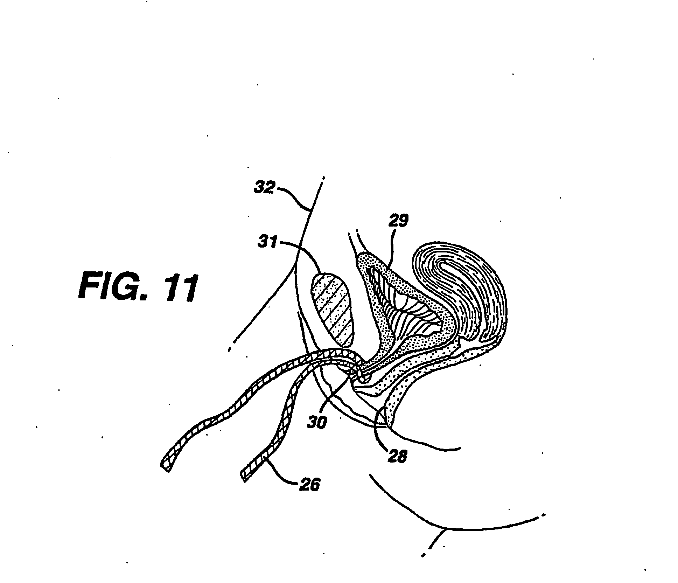

Tape 26 is now located at each side of urethra 30 as shown in FIG. 10 and forming a loop around urethra and located between the urethra and the vaginal wall, Fig. 11. The surplus of the tape at the outside of the abdominal wall is cut off. The tape 26 is left as an implant in the body to form an artificial ligament and provides the support for the urethra as required in order to restore urinary incontinence.

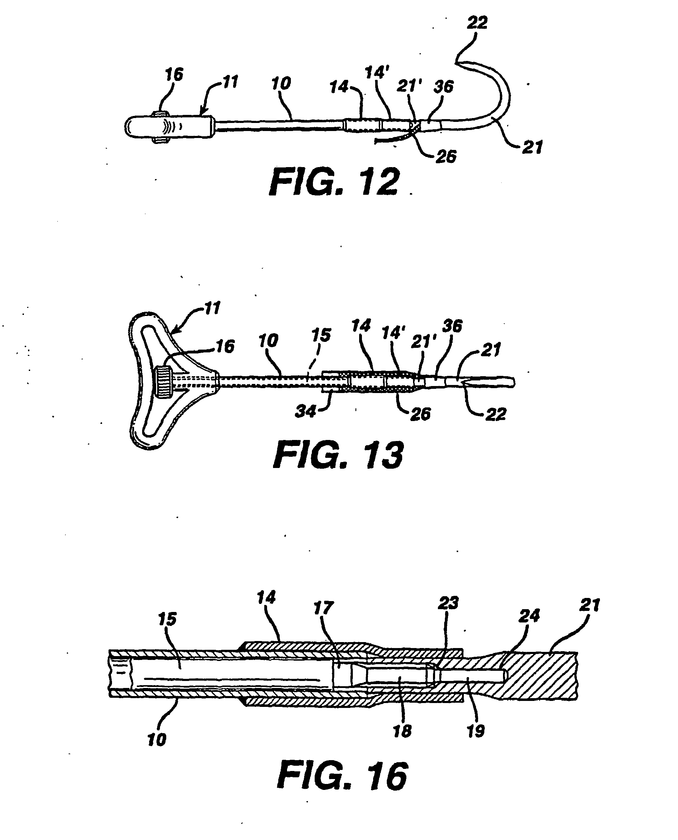

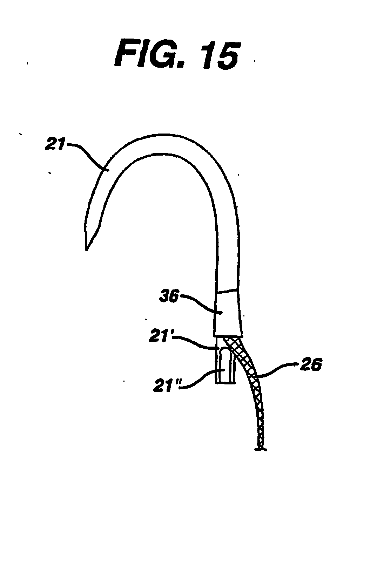

In the embodiment of FIGS. 12 to 17, the end portion 14' of socket 14 is flattened from opposite sides so that the cross section of said end portion is non-circular, and the straight portion 21' of needle 21 at the end to be attached to shank 10 is cylindrical but has milled flat faces 21" over that part of said portion 21', extending from the adjacent end of the needle, which shall be received by socket portion 14'. The predetermined rotational position of the needle in relation to the shank at right angles to the plane of handle 11 is secured by the non-circular shape of socket portion 14' and the end portion of the needle having the flat faces 21", which fits into socket portion 14'. The end portion of the needle having the flat faces 21" joins the body of the needle over a conical portion 33, which tapers towards a shoulder 33'.

In the preferred embodiment, the tape comprises a mesh or netting forming openings of the order of 1 mm. The openings allow fibroblasts to grow into the tape to anchor the tape to surrounding tissue. A suitable material for the tape is PROLENE®, a knitted polypropylene mesh having a thickness of 0.7 mm, manufactured by Ethicon, Inc., Sommerville, New Jersey, USA. This material is approved by FDA in USA for implantation into the human body.

Another kind of tape which may be used in the method can be knitted or woven more closely than the tape mentioned above and can be of such material that the tape after a shorter or longer period will be completely resorbed. By the development of fibroblast proliferation stimulated by the tape reinforcement of the tissue required in order to restore the urinary continence will be obtained.

The material of the tape can be coated with a fibroblast stimulating substance, e.g. an enamel matrix derivative.

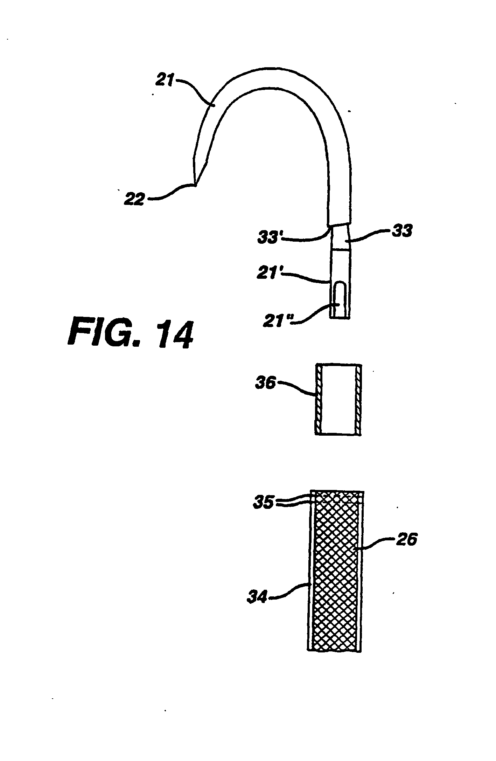

The netting (tape) preferably has a width of approximately 10 mm and is enclosed in a thin polyethylene sheath 34 which in flattened condition has substantially the same width as the tape although a difference in width is shown in FIG 14 for clarity of description purposes. The length of the netting should be approximately 400 mm. The netting and the sheath may be interconnected by of means of two rows 35 of stitching as shown although this is not necessary. The end portion of the sheath is attached to the conical portion 33 of the needle by means of a shrink hose 36 of rubber which extends from the shoulder 331 over the conical portion 33 and partly over the cylindrical end portion 211 of the needle. The shrink hose is substantially flush with the surface of the needle at the shoulder. By this arrangement the netting is securely attached to the needle but if desired the connection can be supplemented by giuing the sheath to portion 33.

The purpose of sheath 34 is above all to facilitate the insertion of the netting in the manner described above i e when the netting is pulled at the ends thereof from the vaginal wall to the abdominal skin and to avoid that rough edges of the netting irritate or damage the body tissues.

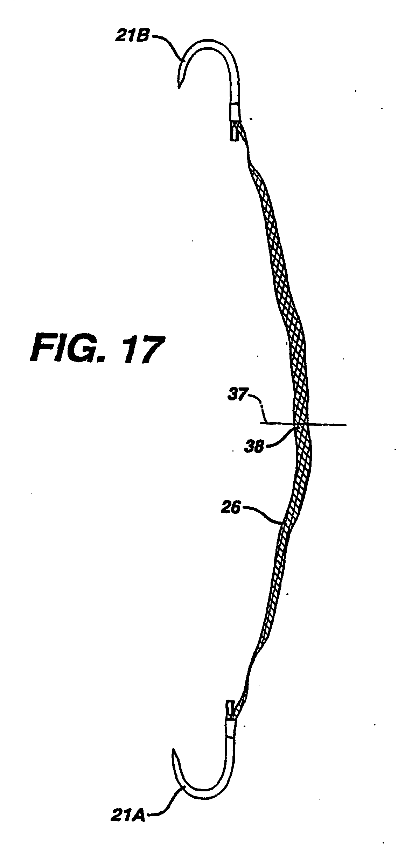

When the tape has been positioned in the correct position as a sling around the urethra the polyethylene sheath shall be removed. In order to facilitate the removal the sheath, the sheath can be perforated at the longitudinal center thereof as indicated by a dot-and-dash line 37 in FIG. 17. The two halves of the sheath can be withdrawn from the body by pulling at the respective outer ends thereof the halves being separated at the perforation under the influence of the pulling force. As an alternative, the sheath can be made in two halves that overlap each other without being interconnected at the longitudinal center of the netting.

The purpose of the polyethylene sheath is also to protect the netting during attachment to the needles and during handling before and during insertion into the body.

The longitudinal center of the tape and sheath should be indicated by a visible color mark 38, FIG. 17 so that the surgeon readily can see when the netting is symmetrically located with reference to urethra during the surgery.

It will be apparent from the foregoing that, while particular forms of the invention have been illustrated and described, various modifications can be made without departing from and scope of the invention. Accordingly, it is not intended that the invention be limited, except as by the appended claims.

## Claims

1. A Surgical instrument for treating female urinary incontinence, comprising:
a) a tape (26) for implanting into the lower abdomen of a female to provide support to the urethra (30); and
b) a curved needle-like element (21) having a proximal end and a distal end and attached to the tape (26); **characterised by** the needle-like element (21) being curved over substantially a half of a circle to pass the tape from an access in the vaginal wall (28), under the pubic bone (31) and to the outside of the abdominal wall (32).

2. The surgical instrument of claim 1 wherein said needle-like element (21) tapers towards the distal end.

3. The surgical instrument of claim 1 or claim 2 wherein the distal end is pointed.

4. The surgical instrument of claim 1 or claim 2 wherein the distal end is blunt.

5. The surgical instrument of any one of claims 1 to 4 wherein the tape (26) is perforated for growth of fibroblasts thereinto.

6. The surgical instrument of any one of claims 1 to 5 wherein the tape (26) is coated with a fibroblast stimulating material.

7. The surgical instrument of any one of claims 1 to 6 wherein the tape (26) is made of polypropylene.

8. The surgical instrument of any one of claims 1 to 7 wherein the tape (26) comprises a netting.

9. The surgical instrument of any one of claims 1 to 8, further comprising a thin plastic sheath (34) enclosing the tape (26).

10. The surgical instrument of claim 9 wherein said sheath (34) is made of polyethylene.

11. The surgical instrument of claim 9 or claim 10 wherein the sheath (34) has a perforation line at the longitudinal center thereof.

12. The surgical instrument of claim 9 or claim 10 wherein the sheath (34) comprises two halves having adjacent ends overlapping each other.

13. The surgical instrument of any one of claims 9 to 12 wherein a visible marking is provided on the sheath (34) at the longitudinal center thereof.

14. The surgical instrument of any one of claims 1 to 13, wherein the needle-like element (21) is detachable from the tape (26) by cutting the tape (26).

15. The surgical instrument of any one of claims 1 to 14, further comprising an attachment mechanism for removably attaching the needle-like element (21) to the tape (26).

16. The surgical instrument of claim 15, wherein the attachment mechanism includes an eye (27) on the needle-like element (21), the tape (26) being passed through the eye (27) to effect the connection.

## Patentansprüche

1. Operationsinstrument zur Behandlung der weiblichen Harninkontinenz, aufweisend:
a) ein Band (26) zum Implantieren in das untere Abdomen einer Frau zur Bereitstellung einer Unterstützung für die Urethra (3 0); und
b) ein gebogenes nadelförmiges Element (21) mit einem proximalen Ende und einem distalen Ende, und das an dem Band (26) befestigt ist; **dadurch gekennzeichnet, dass** das nadelförmige Element (21) im Wesentlichen über eine Hälfte eines Kreises gebogen ist, um das Band von einem Zugang in der Vaginalwand (28) unter dem Schambein (31) und zur Außenseite der Bauchdecke (32) zu führen.

2. Operationsinstrument nach Anspruch 1, wobei das nadelförmige Element (21) sich zum distalen Ende hin verjüngt.

3. Operationsinstrument nach Anspruch 1 oder 2, wobei das distale Ende spitz ist.

4. Operationsinstrument nach Anspruch 1 oder 2, wobei das distale Ende stumpf ist.

5. Operationsinstrument nach einem der Ansprüche 1 bis 4, wobei das Band (26) für ein Wachstum von Fibroblasten perforiert ist.

6. Operationsinstrument nach einem der Ansprüche 1 bis 5, wobei das Band (26) mit einem Fibroblasten stimulierenden Material beschichtet ist.

7. Operationsinstrument nach einem der Ansprüche 1 bis 6, wobei das Band (26) aus Polypropylen hergestellt ist.

8. Operationsinstrument nach einem der Ansprüche 1 bis 7, wobei das Band (26) ein Geflecht umfasst.

9. Operationsinstrument nach einem der Ansprüche 1 bis 8, das ferner eine dünne Plastikhülle (34) aufweist, die das Band (26) umhüllt.

10. Operationsinstrument nach Anspruch 9, wobei die Hülle (34) aus Polyethylen hergestellt ist.

11. Operationsinstrument nach Anspruch 9 oder 10, wobei die Hülle (34) eine Perforationslinie an der Längsachse derselben aufweist.

12. Operationsinstrument nach Anspruch 9 oder 10, wobei die Hülle (34) zwei Hälften mit benachbarten Enden aufweist, die sich gegenseitig überlappen.

13. Operationsinstrument nach einem der Ansprüche 9 bis 12, wobei eine sichtbare Markierung auf der Hülle (34) an der Längsachse derselben vorgesehen ist.

14. Operationsinstrument nach einem der Ansprüche 1 bis 13, wobei das nadelförmige Element (21) von dem Band (26) durch Schneiden des Bands (26) trennbar ist.

15. Operationsinstrument nach einem der Ansprüche 1 bis 14, das ferner einen Befestigungsmechanismus zum lösbaren Befestigen des nadelförmigen Elements (21) an dem Band (26) aufweist.

16. Operationsinstrument nach Anspruch 15, wobei der Befestigungsmechanismus ein Öhr (27) auf dem nadelförmigen Element (21) aufweist, wobei das Band (26) durch das Öhr (27) geführt wird, um die Verbindung zu bewirken.

## Revendications

1. Instrument chirurgical pour traiter l'incontinence urinaire féminine, comprenant :
a) une bande (26) destinée à être implantée dans le bas ventre d'une femme pour fournir le maintien à l'urètre (30) ; et
b) un élément incurvé en forme d'aiguille (21) ayant une extrémité proximale et une extrémité distale et fixé à la bande (26), **caractérisé par** l'élément en forme d'aiguille (21) qui est incurvé sensiblement sur une moitié d'un cercle pour faire passer la bande à partir d'un accès dans la paroi vaginale (28), sous l'os pubien (31) et jusqu'à l'extérieur de la paroi abdominale (32).

2. Instrument chirurgical selon la revendication 1, dans lequel ledit élément en forme d'aiguille (21) se rétrécit progressivement vers l'extrémité distale.

3. Instrument chirurgical selon la revendication 1 ou la revendication 2, dans lequel l'extrémité distale est pointue.

4. Instrument chirurgical selon la revendication 1 ou la revendication 2, dans lequel l'extrémité distale est émoussée.

5. Instrument chirurgical selon l'une quelconque des revendications 1 à 4, dans lequel la bande (26) est perforée pour le développement des fibroblastes à l'intérieur de celle-ci.

6. Instrument chirurgical selon l'une quelconque des revendications 1 à 5, dans lequel la bande (26) est recouverte avec un matériau de stimulation des fibroblastes.

7. Instrument chirurgical selon l'une quelconque des revendications 1 à 6, dans lequel la bande (26) est réalisée à partir de polypropylène.

8. Instrument chirurgical selon l'une quelconque des revendications 1 à 7, dans lequel la bande (26) comprend un filet.

9. Instrument chirurgical selon l'une quelconque des revendications 1 à 8, comprenant en outre une fine gaine en plastique (34) enfermant la bande (26).

10. Instrument chirurgical selon la revendication 9, dans lequel ladite gaine (34) est réalisée à partir de polyéthylène.

11. Instrument chirurgical selon la revendication 9 ou la revendication 10, dans lequel la gaine (34) a une ligne de perforations au niveau de son centre longitudinal.

12. Instrument chirurgical selon la revendication 9 ou la revendication 10, dans lequel la gaine (34) comprend deux moitiés ayant des extrémités adjacentes qui se chevauchent.

13. Instrument chirurgical selon l'une quelconque des revendications 9 à 12, dans lequel une marque visible est prévue sur la gaine (34) au niveau de son centre longitudinal.

14. Instrument chirurgical selon l'une quelconque des revendications 1 à 13, dans lequel l'élément en forme d'aiguille (21) est détachable de la bande (26) en coupant la bande (26).

15. Instrument chirurgical selon l'une quelconque des revendications 1 à 14, comprenant en outre un mécanisme de fixation pour fixer de manière amovible l'élément en forme d'aiguille (21) à la bande (26).

16. Instrument chirurgical selon la revendication 15, dans lequel le mécanisme de fixation comprend un oeillet (27) sur l'élément en forme d'aiguille (21), la bande (26) étant passée à travers l'oeil-let (27) pour effectuer le raccordement.
